(19) Europäisches Patentamt
European Patent Office
Office européen des brevets

(11) **EP 3 014 260 B1**

(12) **EUROPEAN PATENT SPECIFICATION**

(45) Date of publication and mention of the grant of the patent:
**15.05.2019 Bulletin 2019/20**

(21) Application number: **14732626.8**

(22) Date of filing: **23.06.2014**

(51) Int Cl.:
*G01N 29/06* (2006.01)     *A61B 8/13* (2006.01)
*A61B 5/00* (2006.01)     *G01N 29/24* (2006.01)

(86) International application number:
**PCT/GB2014/051910**

(87) International publication number:
**WO 2014/207440 (31.12.2014 Gazette 2014/53)**

(54) **APPARATUS AND METHOD FOR PERFORMING PHOTOACOUSTIC TOMOGRAPHY**

VORRICHTUNG UND VERFAHREN ZUR DURCHFÜHRUNG EINER PHOTOAKUSTISCHEN TOMOGRAFIE

APPAREIL ET PROCÉDÉ POUR RÉALISER UNE TOMOGRAPHIE PHOTO-ACOUSTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.06.2013 GB 201311314**

(43) Date of publication of application:
**04.05.2016 Bulletin 2016/18**

(73) Proprietor: **UCL Business Plc.**
**London W1T 4TP (GB)**

(72) Inventors:
• **ZHANG, Edward**
  **London W1T 4TP (GB)**
• **BETCKE, Marta**
  **London W1T 4TP (GB)**
• **ARRIDGE, Simon**
  **London W1T 4TP (GB)**
• **BEARD, Paul**
  **London W1T 4TP (GB)**
• **COX, Ben**
  **London W1T 4TP (GB)**

(74) Representative: **D Young & Co LLP**
**120 Holborn**
**London EC1N 2DY (GB)**

(56) References cited:
**US-A- 6 075 603     US-B1- 7 095 505**
**US-B1- 7 262 861**

## Description

### Field of the Invention

The present application relates to a method and apparatus for performing photoacoustic tomography, and in particular to providing such a method and apparatus having a reduced image acquisition time.

### Background of the Invention

Photoacoustic tomography (PAT) is an emerging biomedical imaging modality with both pre-clinical and clinical applications, and can provide complementary information to established imaging techniques [2, 30]. In the majority of such established imaging techniques, either the useful contrast mechanism is of low resolution, or high resolution images that are of limited contrast. PAT is an example of "imaging from coupled physics", a new class of techniques, in which contrast induced by one physical mechanism is read by a different physical mechanism, so that both high resolution and high contrast can be obtained at the same time

In PAT, ultrasound waves are excited by irradiating tissue with modulated electromagnetic radiation from a laser, usually pulsed on a nanosecond timescale. The wavelength of the electromagnetic radiation is typically in the optical or near infrared band. In general, the longer wavelengths allow greater penetration of the radiation into the tissue, of the order of several centimetres. The radiation is then absorbed within the tissue and leads to a slight rise in temperature, typically about 0.1K, which is sufficiently small to avoid any physical damage or physiological change to the tissue. The rise in temperature causes the emission of ultrasound (acoustic) waves, which are broadband (approximately tens of MHz) and relatively low amplitude (less than 10 kPa). These ultrasound waves propagate to the surface of the tissue, where they can be detected by a suitable mechanism, for example, a single mechanically scanned ultrasound receiver or an array of receivers. A PAT image is then (re)constructed from the received ultrasound signals based on the determined travel time and an assumed speed of sound propagation through the tissue.

From a theoretical perspective, the forward problem in PAT describes the acoustic propagation of the initial pressure:

$$p_0(\mathbf{r}) \rightarrow y(\mathbf{m},t) = \Lambda(c,\mu)p_0(\mathbf{r}), \quad \text{Equation (1)}$$

where $\mathbf{r}$ is a position vector, $p_0(\mathbf{r})$ is the pressure at location $\mathbf{r}$ at time $t$=0, $\Lambda$ is the Green's operator of the wave equation, which in general depends on the sound speed $c(\mathbf{r})$ and attenuation $\mu(\mathbf{r})$, both of which are potentially dependent on location $\mathbf{r}$, and $y(\mathbf{m},t)=p(\mathbf{r},t)|_{\mathbf{r}=\mathbf{m}}$ is the time series data recorded at a detector. The inverse problem is to recover the initial pressure $p_0$ from the time series data, thereby effectively reconstructing an image which reflects the distribution of absorption of the excitation radiation.

Many approaches are available for performing this image reconstruction, including analytical methods based on the *Spherical Mean Radon Transform* (SMRT), which require the assumption of uniform sound speed, and *time reversal* methods, which are able to accommodate tissue-realistic acoustic attenuation and heterogeneous sound speed. Following reconstruction, the photoacoustic image can be used as an input to a second inversion step known as *Quantitative Photoacoustic Tomography* (QPAT), which outputs spatial maps of concentrations of chromophores corresponding to physiological components.

An important difference between PAT and conventional ultrasound (US) is that in the latter, the image contrast is determined by differences in acoustic impedance - which are frequently not that great between different tissue types. In contrast, in PAT the differences in optical absorption between different tissue types can be much greater, thereby providing very good image contrast. As an example, haemoglobin exhibits strong preferential optical absorption, hence PAT can provide very detailed images of microvasculature (which are difficult to obtain using conventional ultrasound, due to weak echogenicity). In addition, the exciting laser radiation may be tuned to a specific wavelength, for example, to correspond to an absorption peak of a particular substance. By acquiring images at multiple different wavelengths, PAT is able to map the distribution of various biochemicals in great detail.

The growth of interest in PAT in the last decade has been rapid, and it has been used to visualise, inter alia, small animal anatomy, pathology and physiology, murine cerebral vasculature [31], capillary beds *in vivo* [33], implanted subcutaneous tumours [16], [33], murine embyros [15], and *in vivo* atherosclerosis [1]. As noted above, PAT can be used spectroscopically for molecular and genomic imaging, differentiating endogenous chromophores or contrast agents according to their absorption spectra, and also for functional imaging, such as measuring changes in blood oxygenation and flow. High frame rate (>10Hz) photoacoustic imaging systems have been demonstrated using curved and linear piezoelectric arrays, but they are usually limited to 2D (planar) imaging, rather than 3D (volume) imaging. Further information about PAT can be found in [34].

At UCL, an ultrasound detection array has been developed based on a Fabry-Perot interferometer for use in PAT - see "Backward-mode multi-wavelength photoacoustic scanner using a planar Fabry-Perot polymer film ultrasound sensor for high-resolution three-dimensional imaging of biological tissues" by Zhang et al, Applied Optics, 47, 4, 561-577, 1 February 2008. This Fabry-Perot interferometer detector can record acoustic pressure with very small element sizes while retaining high sensitivity, thereby generating high quality, high resolution three-dimensional photoacoustic images (something that is difficult, if not impossible, with conventional arrays of piezo-electric sensors for detecting ultrasound).

However, a significant bottleneck in state of the art high resolution PAT systems is the data acquisition time. For example, in the Fabry-Perot interferometer detector mentioned above, the acquisition time for a static image is at least several minutes. At present therefore, such high resolution PAT systems are too slow for effective real-time or dynamic imaging of physiological processes.

US 6075603 discloses a contactless system for imaging an acoustic source within a workpiece, which directs a preferably annular optical probe beam pattern onto the vibrating workpiece surface, with the vibrationally modulated beam then detected by an array of preferably non-steady-state photo-emf detectors arranged in a similar pattern. The probe beam is scanned over the vibrating surface, either mechanically or through an electronically simulated phased array scheme. Time gating is used to suppress unwanted side-lobes when the individual detector outputs are summed over an appreciable waveband. A self-calibration scheme is also preferably used that provides a quantitative as well as qualitative output. A calibration modulation is imposed on at least one of the probe and reference beams, with the calibration modulation later removed at a post-detector stage and used to normalize the acoustic modulation output. Variations of the self-calibration scheme include reference-beam and time-delay interferometers based upon a calibrating phase modulation, and a photon flux measurement approach based upon a calibrating amplitude modulation.

US 7262861 discloses a laser ultrasonic inspection apparatus and method which enables remote sensing of thickness, hardness, temperature and/or internal defect detection is disclosed. A laser generator impinges a workpiece with light for generating a thermo-elastic acoustic reaction in a workpiece. A probe laser impinges the workpiece with an annularly-shaped probe light for interaction with the acoustic signal in the workpiece resulting in a modulated return beam. A photodetector having a sensitive region for detecting an annularly-shaped fringe pattern generated by an interaction of a reference signal and with the modulated return beam at said sensitive region.

## Summary of the Invention

The invention is defined in the appended claims.

The approach described herein provides compressed measurement acquisition in PAT, whereby encoding with patterns incoherent to the pressure at all time steps is used to form a compressed measurement. A considerably lower number of compressed measurements than regular measurements is needed for exact reconstruction under a hypothesis of compressibility of the data/PAT image in an *a priori* chosen basis. Such an approach helps to support a PAT system which allows fast dynamic imaging of time-varying physiological processes, for example, functional studies of blood flow and oxygen saturation changes, investigations of trauma response and microcirculation, and pharmacokinetics, such as drug uptake and perfusion.

## Brief Description of the Drawings

Various embodiments of the invention will now be described in detail by way of example only with reference to the following drawings:

Figure 1 is a schematic diagram of a known apparatus for PAT;

Figure 2 is a schematic diagram of an apparatus for performing PAT in accordance with an embodiment of the invention;

Figure 3 shows two examples of mask patterns for use with the apparatus of Figure 2 (for simplicity, the number of pixels per mask pattern is reduced in Figure 3).

Figure 4 is a simplified flowchart of a method for obtaining a PAT image from data acquired using the apparatus of Figure 2 in accordance with an embodiment of the invention.

Figure 5 is an example of image reconstruction from compressed measurements.

Figures 6 and 7 provide schematic illustrations of the dynamic reconstruction of an image acquired by compressed sensing.

Figure 8 is an example of variation in sensitivity for a given wavelength across the surface of an FP interferometer detector such as included in the apparatus of Figures 1 and 2.

**Detailed Description**

In PAT a short (ns) pulse of near-infrared or visible light is sent into tissue, whereupon absorption of photons generates a small local increase in pressure which propagates to the surface as a broadband ultrasound pulse. The amplitude of this signal is recorded at the tissue surface, such as by an array of sensors, and an image reconstruction algorithm is employed to estimate the original 3D pressure increase due to optical absorption - this is the photoacoustic image.

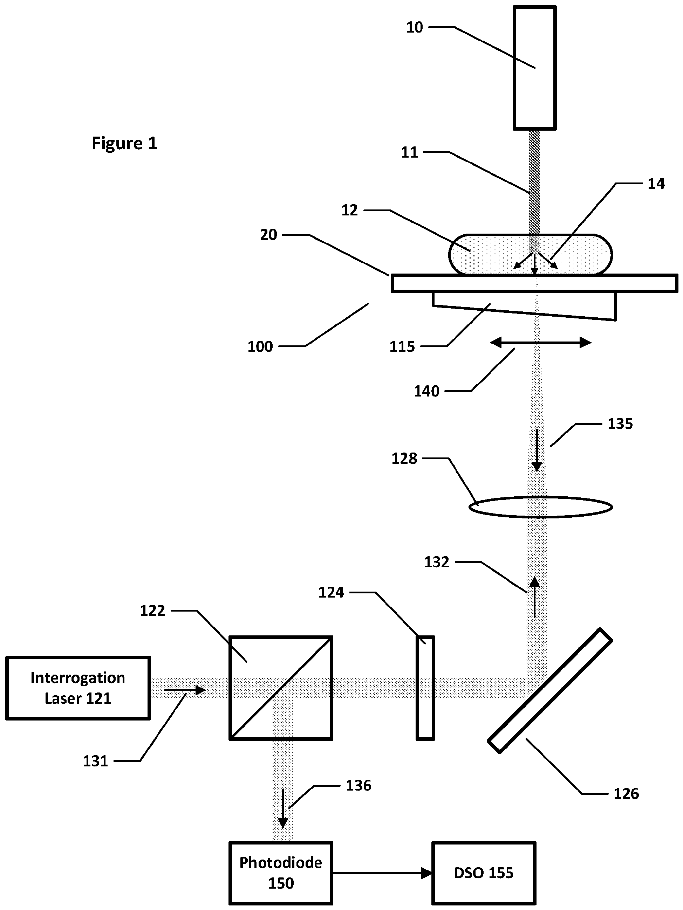

Figure 1 is a schematic diagram of a PAT system including a Fabry-Perot interferometer detector 20 as described in the above-referenced paper by Zhang et al.. A sample of tissue 12 being imaged is subjected to an excitation beam 11 comprising a pulse of electromagnetic radiation (optical, near infrared, or microwave) from excitation laser 10, which may be a multiple wavelength laser or a single wavelength laser. The output from excitation laser 10 typically falls within the wavelength range 400-2000 nm (although microwaves might also be used). The electromagnetic radiation 11 is absorbed within the tissue 12, resulting in the generation of ultrasound waves 14 which propagate from the absorption locations to the surface of tissue sample 12.

The tissue sample 12 rests on (is in acoustic contact with) a sensor head 100 comprising a substrate 115 on which is formed a Fabry-Perot (FP) interferometer detector 20. As the ultrasound waves 14 reach the surface of the tissue sample 12, they cause corresponding changes in the optical thickness of the Fabry-Perot interferometer detector 20 at the locations where the ultrasound waves exit the tissue sample, thereby allowing the Fabry-Perot interferometer detector 20 to detect these ultrasound waves. Conceptually, we can regard the acoustic wave pressure as forming an image which is detected using the sensor head 100. More particularly, the sensor head 100 produces, for each monitored location of the FP detector 20, a time series representing the arrival of ultrasound waves arising from the excitation pulse at that particular location.

The Fabry-Perot interferometer detector 20 and its operation are described in detail in the above-referenced paper by Zhang et al. -we present here a high-level and somewhat simplified description (for full details, please see the cited paper). The Fabry-Perot interferometer detector 20 utilises an interrogation laser 121 which generates an interrogation optical beam 131, for example at a wavelength of 1550 nm, which is used in conjunction with sensor head 100 for 'reading' the image formed on the FP detector. The optical beam 131 is directed to a beam-splitter 122, from where it passes through a quarter wavelength ($\lambda$/4) plate 124 onto a scanning mirror 126 (described in more detail below). The scanning mirror 126 directs the interrogation beam through an objective (convex) lens 128 to form an incident interrogation beam 132 which is focussed onto the underside of the sensor head 100. The sensor head 100 functions, in effect, as a thin film Fabry-Perot cavity, whereby a first portion of the incident beam is reflected from a first (lower) surface of the FP detector 20, while a second portion of the incident beam passes further into the FP detector 20 and is reflected from a second (upper and opposing) surface of the FP detector 20. The first and second reflected portions re-combine, and interfere with one another, to form a reflected interrogation beam 135 (more accurately, the interference occurs between multiple reflections within the sensor head). The reflected beam 135 returns along the path of the incident beam 132, travelling back from the sensor head 100 through the objective lens 128, the scanning mirror 126 and the quarter wavelength ($\lambda$/4) plate 124 to the beam splitter 122. The two passages through plate 124 rotate the polarisation state by 90 degrees, relative to the original beam 131, hence the reflected interrogation beam 135 is directed by the beam splitter 122, as indicated by arrow 136, onto photodetector (photodiode) 150, which is connected to a digitizing oscilloscope (DSO) 155 (and from there to a computer, not shown in Figure 1).

The scanning mirror 126 is used to build up an image of the sensor head 100 by sequentially directing the incident interrogation beam 132 to different locations on the sensor head 100, as indicated by arrow 140. In particular, by suitable displacements and/or rotations of the scanning mirror 126, the incident interrogation beam 132 steps across the two-dimensional underside of the sensor 100 in order to 'read' an ultrasound image produced by acoustic waves 14 on the FP interferometer detector 20.

In the absence of an excitation beam 11, the resulting image acquired by scanning the interrogation beam 132 across the sensor head generally includes sets of fringes, which arise from slow variations in the thickness across the sensor head, and hence in the amount of interference between the first and second reflected portions (as noted above, more accurately, the interference occurs between multiple reflections within the sensor head). In the presence of the excitation beam 11, the ultrasound waves 14 induce an acoustic modulation of the optical thickness of the sensor head 100, which produces a small additional phase shift between the first and second reflected portions. When the interrogation wavelength is set to the peak derivative of the interferometer transfer function, which represents the reflected intensity versus wavelength, this additional phase shift is linearly converted, in accordance with the transfer function of the interferometer 20, into a corresponding modulation of the reflected optical power, which can be picked up by the photodiode 150 and DSO 155. Accordingly, for each scanning position, a time series representing the acoustic ultrasound signal at that location is obtained.

The acquired data set is therefore three-dimensional (two spatial, one temporal) and provides $y(\mathbf{m},t)$ from Equation (1) above, where $\mathbf{m}$ represents an x,y location in the plane corresponding to the FP interferometer detector 20. As part of the image reconstruction process, the value of the initial pressure distribution is first recovered, $p_0(\mathbf{r})$, (by inverting Equation (1)), to form a three-dimensional PAT image. This recovered image may be used as the initial step of a second reconstruction problem, Quantitative Photoacoustic Tomography (QPAT), whose aim is to quantitatively represent the three-dimensional (spatial) distribution of chromophores within tissue 12. The final output of this image reconstruction is therefore a single three-dimensional image at time $t$=0, i.e. when $p$= $p_0$. In other words, timing information in the acquired data $y(\mathbf{m},t)$ does not reflect changes in the sample with time (it is not a dynamic image), but rather the travel time of the acoustic signal since t=0, which in turn reflects the distance that the signal has travelled (and also the speed of sound along that path). If we consider a simplified model in which the acoustic signal just propagates in a z direction, perpendicular to the FP interferometer 20 and sensor head 100, which have a substantially two-dimensional (x-y) geometry, then the spatial information in $y(\mathbf{m},t)$ would represent a projection of the three-dimensional sample onto the planar geometry of the sensor head 100, and the timing information would then encode travel time data for the acoustic signal to provide information directly in respect of the third spatial dimension (z). However, since the acoustic signal travels spherically outwards (rather than just along the z-axis), this in effect mixes the coordinates, so that the signal $y(\mathbf{m},t)$ originates from a surface in the sample 12 defined by the radial distance ct where t is the travel time from $\mathbf{m}$ and c is the propagation speed.

In the implementation of Zhang et al., the acquisition time is approximately 1 second per scan step. This acquisition time is dominated by the time taken to rearm the DSO 155 between acquisitions, and also the download time from the DSO 155 to the computer (although it is suggested that the latter could be reduced by providing the DSO with an on-board memory to buffer results across different scan locations). Having an acquisition time of 1 second per scanning step results in an overall acquisition time of 1 hour for a 60x60 pixel image - which is a rather small image size. The overall acquisition time for a 600x600 pixel image would be 100 hours or four days, so this is a lengthy procedure and is clearly much too slow to investigate *in vivo* dynamic processes.

In general terms, the ultimate limit to the data acquisition rate in PAT is set by the propagation time for the (ultra)sound to cross the specimen. As an example, if a sample has a depth of 15mm, the propagation time is approximately 10 $\mu$s, giving a maximum data acquisition rate of 100 kHz. One practical limitation is the rate at which the laser 10 can be pulsed. As an example of this limitation, currently available laser systems that produce a single wavelength pulse of sufficient power for PAT typically have a maximum pulse repetition rate of 1000 Hz. If the system of Figure 1 could operate at this rate, then a 600x600 image could be produced in approximately 6 minutes (360 seconds). However, this is still significantly too slow to image dynamic physiological changes, for which a frame rate of at least 1 Hz (i.e. a *frame acquisition time* of < 1 second) is generally required.

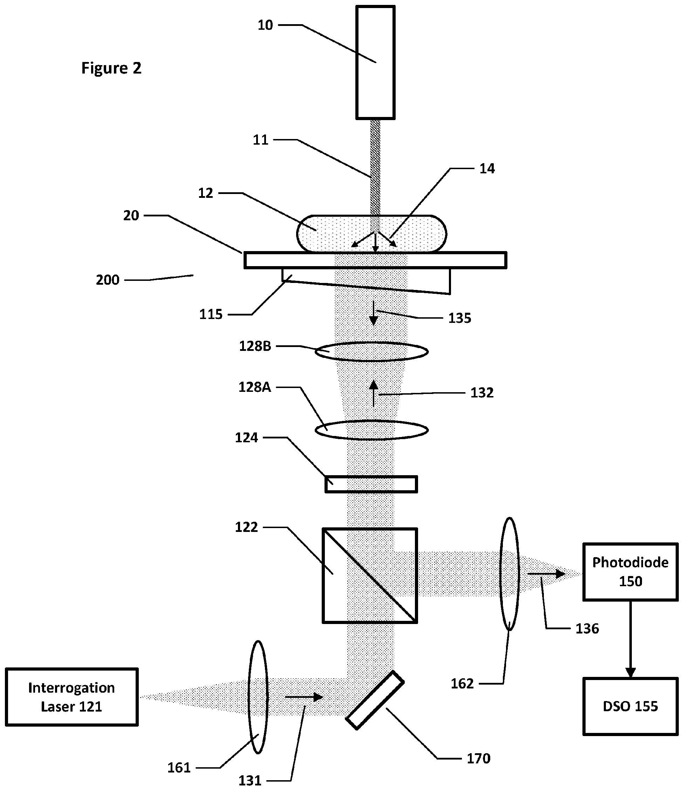

Figure 2 is a schematic diagram of a PAT system in accordance with an embodiment of the invention which is a modification of the system shown in Figure 1. In general, components in the embodiment of Figure 2 which are (at least substantially) the same as the corresponding components in the embodiment of Figure 1 are denoted by matching reference numerals. Please refer to the description of Figure 1 above for further information about these corresponding components.

In Figure 2 (as in Figure 1), a sample of tissue 12 being imaged is subjected to an excitation beam 11 comprising a pulse of electromagnetic radiation (optical or near infrared or microwave) from excitation laser 10, which may be a multiple wavelength laser or a single wavelength laser. The electromagnetic radiation 11 is absorbed within the tissue 12, resulting in the generation of ultrasound waves 14 which propagate from the absorption locations to the surface of tissue sample 12.

The tissue sample 12 rests on (is in acoustic contact with) a sensor head 200 comprising a substrate 115 on which is formed a Fabry-Perot interferometer detector 20. As the ultrasound waves 14 reach the surface of the tissue sample 12, they cause corresponding changes in the optical thickness of the Fabry-Perot interferometer detector 20 at the locations where the ultrasound waves exit the tissue sample, thereby allowing the Fabry-Perot interferometer detector 20 to detect these changes. Although the sensor head 200 in Figure 2 is the same as or similar to the sensor head 100 in Figure 1, there are significant differences between Figure 1 and Figure 2 in terms of how the apparatus reads (interrogates) the sensor head 200.

The apparatus of Figure 2 includes an interrogation laser 121 which generates an interrogation optical beam 131 for use in conjunction with the sensor head 100 for reading the image produced by the FP interferometer detector 20. In contrast to the system of Figure 1, the interrogation optical beam is formed to have a relatively broad cross-section in the plane perpendicular to the direction of propagation of the beam. The broader cross-section is formed using convex lens 161 (or any other suitable set of optical components).

The interrogation optical beam 131 is then reflected by a micromirror array 170, which is described in more detail below. Note that the interrogation optical beam 131 may have a cross-section to match the whole active surface of micromirror array 170. The optical beam 131 is reflected by micromirror array 170 to a beam-splitter 122, from where it passes through a quarter wavelength ($\lambda$/4) plate 124 as for the configuration of Figure 1. A pair of objective lenses 128A and 128B is located between the quarter wavelength plate 124 and the sensor head 200. This pair of objective lenses 128A and 128B (or any other suitable set of optical components) increases the cross-section of the incident interrogation optical beam 132 from the size as determined by the micromirror array 170 to a size corresponding to the desired (physical) size of the image to be obtained. Accordingly, the same micromirror array 170 can be used with different sizes of sample 12 and/or different sizes of sensor head 100.

As described above, the sensor head 200 functions as a thin film Fabry-Perot cavity, whereby a first portion of the incident beam 132 is reflected from the first (lower) surface of the FP interferometer detector 20, while a second portion of the incident beam 132 passes into the FP interferometer detector 20 and is reflected from the second (upper) surface of the FP interferometer detector 20. The first and second (and subsequent) reflected portions then re-combine and interfere with one another, to form a reflected interrogation beam 135. The reflected beam 135 returns along the path of the incident beam 132, travelling back from the sensor head 20 through the objective lenses 128B, 128A and the quarter wavelength ($\lambda/4$) plate 124 to the beam splitter 122. The two passages through plate 124 rotate the polarisation state by 90 degrees relative to the original beam 131, hence the reflected interrogation beam 135 is directed by the beam splitter 122 to a convex lens 162 which focusses the (relatively broad) reflected interrogation beam, as indicated by arrow 136, onto photodetector (photodiode) 150. This photodiode is connected to a digitizing oscilloscope (DSO) 155 and from there to a computer (not shown), as per the configuration of Figure 1.

As for the system of Figure 1, in the presence of the excitation beam 11, the ultrasound waves 14 induce an acoustic modulation of the optical thickness of the FP interferometer detector 20, which produces a small additional phase shift between the first and subsequent reflected portions. This additional phase shift is linearly converted, in accordance with the transfer function of the interferometer 20, into a corresponding modulation of the reflected optical power, which can be picked up by the photodiode 150 and DSO 155. However, in contrast to the system of Figure 1, which builds up an overall image of the photoacoustic waves by sequentially scanning the incident interrogation beam 132 across different locations on the sensor head 200, the system of Figure 2 effectively interrogates the ***complete*** image provided by the sensor head 200.

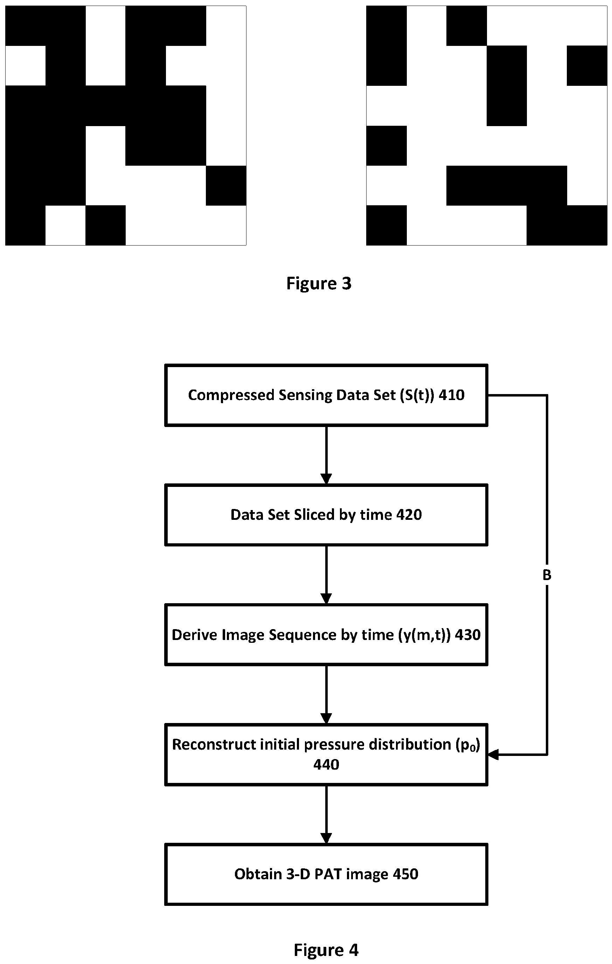

Figure 3 illustrates two example mask patterns, which for ease of representation are 6x6 patterns comprising a set of binary pixel values. The actual mask patterns used in conjunction with the embodiment of Figure 2 have many more pixels (as described below) to give better image resolution, and comprise random patterns drawn from, for example, Bernoulli/Gaussian distribution [7]. In addition, the mask patterns shown in Figure 3 are binary masks, with pixel values of 0 or 1, as are the mask patterns used in conjunction with the embodiment of Figure 2, but greyscale masks having intermediate values can also be used.

The output, as detected by photodiode 150 of Figure 2, represents a spatial integral of the image signal produced by the sensor head 20 weighted according to the given mask pattern applied to the micromirror array 170. In other words, in the case of a binary mask, the output effectively adds together the values of the regions of the image signal produced by the sensor head 200 in which the mask is reflective (a value of one) and ignores the other regions of the image signal for which the mask is non-reflective (a value of zero). Therefore each mask pattern results in a corresponding output detected at the photodiode. By utilising a sequence of different mask patterns, and obtaining a corresponding sequence of output values from the photodiode 150, a data set is acquired, from which the overall image produced by the Fabry-Perot interferometer detector 20 can be reconstructed, as well as the initial pressure distribution ($p_0$).

More particularly, the output data signal from the system of Figure 2 is a set of time series $s_i(t)$, where $i=1 \ldots$ N is an index to identify the mask pattern $M_i$ used for that time series. We can relate $s_i(t)$ to the signal $y(\mathbf{m},t)$ received at the FP interferometer detector 20 (and which is measured directly with the apparatus of Figure 1), as follows:

$$s_i(t) = \sum_{x,y} y(\mathbf{m}(x,y),t) \times M_i(x,y), \quad \text{Equation (2)}$$

where x,y represent (i) coordinates on the acoustic sensing plane of defined by the FP interferometer detector 20, and (ii) corresponding coordinates in the plane of a mask pattern such as shown in Figure 3 (as projected onto the sensing plane), and the summation is across the whole area of the sensing plane (upon which the interrogation beam 132 is incident), and wherein $M_i(x,y) = 0$ or 1 for a binary mask such as shown in Figure 3, or more generally $0 \leq M_i(x, y) \leq 1$ for a greyscale mask.

The system of Figure 2 therefore operates in accordance with a technique referred to as compressed sensing. Thus in most image acquisition systems, a spatial distribution of data signals is acquired, for example, using a rectangular array of pixels, where the image is defined by the luminosity (or some other parameter) detected in each pixel in the array. In a typical image, there are significant correlations (redundancy) between the luminosity values of different pixels, which can be exploited by compression algorithms such as JPEG2000. In compressed sensing however, rather than first acquiring a complete image which is later subjected to compression processing, the amount of data acquired in the first place is restricted. It has been found that the number of compressed measurements when performing compressed sensing is generally (significantly) smaller than the number of measurements made in a more conventional system (which does not use compressed sensing/measurements). One example of such an approach is the Rice 'single-pixel' camera [12] which records an image through a small number of random sums of pixels rather than as individual spatial points. More precisely, a signal $y$ in an $N$ dimensional space can be adequately represented in an *a priori* chosen basis $\Psi$ with only a small number of non-zero coefficients, $y= \Psi z$. In compressed sensing, the information of an image is directly measured by sensing a signal $y$ with a properly constructed matrix $A$ with number of rows $M \ll N$ to give a much shorter data vector $s=Ay$. The original signal is then recovered through a solution of the optimisation problem:

$$OP_q: min_z\ J_q(z),\ \text{such that}\ \ ||s-A\Psi z||_2 \leq \delta, \quad \text{Equation (3)}$$

where $J_q(z)=||z||_q$ (length of vector $z$ in the $q$-norm) and $\delta$ allows for measurement noise. Whilst $q=0$ is the 'ideal' measure of sparsity (the number of non-zero elements), the corresponding optimisation problem is classically computational intractable (NP-hard), and so is commonly replaced by a better behaved approximation such as the convex relaxation ($q=1$). Compressed sensing theory then specifies conditions under which the sparsest solution can be robustly recovered by solving ($OP_q$). A variety of specific algorithms are available for solving this problem, including Othogonal Matching Pursuit [27], message passing [11], iteratively reweighted norm [8], and Bregman-type [32] methods. In medical imaging, compressed sensing has been effective, for example, in dynamic MRI [21] and dynamic CT [25]. Compressed sensing for PAT has also been reported, but for quite a different approach from that described herein. In particular, some such existing approaches have used compressed sensing in conjunction with patterned *excitation* light [24], rather than patterned *interrogation* light as described herein. Other existing approaches have recognised PAT images can be represented sparsely in some basis, and then used this understanding to regularise image reconstructions from spatially undersampled data [13, 23]. However, the approach described herein is not directly motivated by such spatial undersampling, but rather provides a way of acquiring compressed measurements (in contrast, compressed sensing may be used to refer to L1 reconstruction, i.e. to the reconstruction of sparse signals, even without taking compressed measurements, as per [13 and 23]).

Returning to Figure 2, in one implementation, the excitation laser 10 is a diode-pumped Q-switched Nd:YAG laser (Ekspla NL 220S, 1 kHz pulse repetition rate) (see www.ekspla.com). The trigger from the excitation laser is used to control switching of the mask patterns, i.e. from one mask pattern to the next mask pattern, by the micromirror array 170. The interrogation laser 121 is a 1490-1640nm high power fibre-coupled tunable laser (Yenista Tunics T100S-CL-WB) (see www.yenista.com) which is used to produce interrogation beam 131 which illuminates the micromirror array 170. A beam shaper (not shown in Figure 2), such as available from Topag Lasertechnik (see www.topag.de), converts the Gaussian beam profile from the interrogation laser 121 into a square top hat profile to provide uniform illumination of the micromirror array 170.

In one implementation, the micromirror array 170 comprises a Digital Micromirror Device 0.7" XGA 2xLVDS DMD from Texas Instruments (see www.ti.com and TI DN 2509929, Rev A, September 2008). This device comprises 1024x768 pixels and is therefore used to apply mask patterns accordingly (as opposed to the simple 6x6 mask patterns shown in Figure 3). The frame rate is up to 22 kHz for binary patterns. The pitch of the micromirrors is 13.7$\mu$m, corresponding to a device size of 14x10.5mm. However, as shown in Figure 2, the interrogation beam 132 reflected from the micromirror array 170 can be scaled by a suitable optical system, such as lens combination 128A, 128B, to provide a desired image scale size on the Fabry-Perot interferometer detector 20. For example, a 2:1 scaling for the interrogation beam 132 gives a pixel scale of 27.4$\mu$m and an overall image size of 28x21mm on sensor device 200. If a larger or denser array is required, two or more micromirror arrays could be used in combination with one another, placed side by side. The micromirror array 170 is controlled by a development platform, Discovery 4100 (see http://www.ti.com/tool/dlpd4x00kit), which presents a USB 2.0 interface to a control computer (not shown in Figure 2) to allow the micromirror array 170 to be programmed with the desired patterns.

In one implementation, the photodetector 150 comprises a balanced photodetector built from matched photodiodes. This allows higher intensities for the interrogation light beam 131, which in turn helps to improve the signal-to-noise ratio of the resulting PAT images. The DSO 155, for example as available from Tektronix (see www.tek.com), samples the output from the photodetector 150 at 250MHz, thereby allowing the full bandwidth of the photoacoustic signal to be detected. The DSO is provided with on-board memory to avoid the need for mid-measurement data downloads (which would interrupt, and hence slow down, the data acquisition process). Note that since each mask pattern only produces a single output sample (representing a compressed measurement), these on-board memory requirements are relatively modest.

Figure 4 is a simplified flowchart of a method for obtaining a PAT image from data acquired using the apparatus of Figure 2 in accordance with an embodiment of the invention. The data set output from the apparatus of Figure 2, as per operation 410 in Figure 4, comprises a set of time series of intensity values from the photodetector 150, each time series corresponding to its respective (known) mask pattern which was applied to the interrogation light beam 131 for obtaining that particular intensity time series (as per Equation 2). We can represent this output as $s(t)=Ay(\boldsymbol{m},t)$, where

A is a $n_{pattern}$ x $n_{pix}$ matrix, where $n_{pattern}$ represents the total number of patterns in the sequence and $n_{pix}$ represents the total number of pixels per mask pattern. Each row of A represents a binary or grayscale pattern of length $n_{pix}$ (Figure 3 depicts binary masks, but grayscale masks could also be utilised). Note that $s(t)$ incorporates the full set of $s_i(t)$ as defined in Equation 2 above, i.e. it includes the output for each mask $M_i$.

In operation 420, the data set is sliced by time. In other words, we collect the data values from each time series (i.e. for each mask pattern) at a specific time. At each such time step t, the data $y(\mathbf{m},t)$ is recoverable from s(t) in accordance with the optimisation of Equation (3) as defined above, as per operation 430. In effect, this recovers the $y(\mathbf{m},t)$ time sample by time sample using the same approach as the Rice camera [12] (because each time step can be considered as representing a single image across the sensor plane which is sampled by the various mask patterns). Given the nature of wave propagation, the optimal sparsifying transformation $\Psi^y$ may be based, for example, on 2D curvlets [5], contourlets [10] or shearlets [20].

Once $y(\mathbf{m},t)$ has been recovered in operation 430, then this can be transformed in operation 440 into a photoacoustic image representing the initial pressure distribution $p_0$, as per the inverse problem from Equation (1), and this initial pressure distribution can then be transformed in operation 450 to a three-dimensional QPAT image showing the distribution of material responsible for the absorption of the excitation radiation. Note that operations 440 and 450 are also employed with respect to the known apparatus of Figure 1, as per the paper by Zhang et al. For example, operation 440 can be performed using a reconstruction algorithm that inverts the spherical mean radon transform (SMRT), while operation 450 can be performed using quantitative photoacoustic tomography (QPAT), as discussed above.

An alternative approach to the operations 410 through to 440 is to recover the initial pressure distribution $p_0$ directly from the measured data from the apparatus of Figure 2, namely s(t), as illustrated schematically by arrow B in Figure 4. This again can be approached through Equation (3), with z now a (3D) spatially compressed representation of the initial pressure (p0) in a basis given by the (3-D) sparsifying transform $\Psi^{p0}$ and the operator modified to $A\Lambda\Psi^{p0}$. The reduction in the number of measurements (i.e. the number of mask patterns to be used), and hence the overall image acquisition time, depends on the incoherence of the resulting sampling matrix $A\Lambda\Psi^{p0}$, which is influenced by the choice of patterns or subsampling scheme and the sparsifying transform $\Psi^{p0}$. 3-D extensions of curvelets and shearlets provide a suitable framework for this approach. In situations where the initial pressure distribution $p_0$ has a sparse representation in the (3D) curvelet frame, the result in [6] implies that both the emitted acoustic wave and the time reversed acoustic field at each time step also have a sparse representation in this basis. Accordingly, a numerical operator can be used for the forward operator $\Lambda$, taking advantage of sparse wave propagation in place of existing Fourier based pseudo-spectral methods.

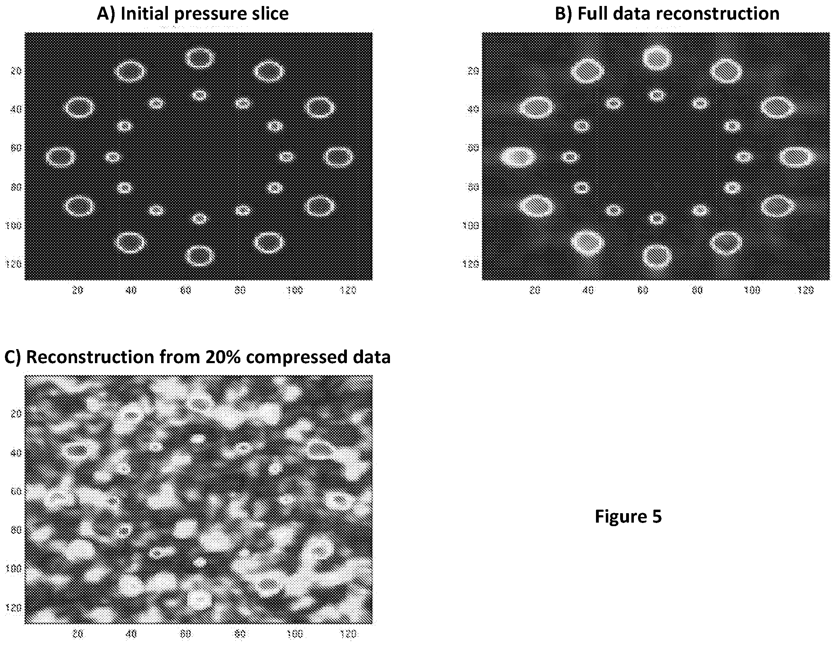

Figure 5 is an example of reconstructing a compressed sensing image (for simulated data) using the method of Figure 4. The simulated data relates to a target volume of 128x128x64 voxels that contains a helical pattern, where the 128x128 voxels can be regarded as corresponding to the X-Y axes, and 64 as the Z (depth) axis. The images are displayed as maximum intensity projections onto the X-Y plane.

Figure 5A depicts the original image distribution in the target volume. Figure 5B shows the result of performing a full image reconstruction, which might be adopted, for example, by acquiring the image data using the apparatus shown in Figure 1. Figure 5C shows the result of performing an image reconstruction from data obtained using compressed sensing in accordance with an embodiment of the invention, for example, by acquiring the image data using the apparatus shown in Figure 2. The acquired data for Figure 5C is compressed to about 20% of the acquired data for Figure 5B. It can be seen that this leads to increased noise in the resulting reconstructed image, but the main features, including smaller objects, remain visible. In addition, since the noise in Figure 5 is generally random over time, it can be suppressed, for example, by averaging across a number of images. It is likely that the use of L1 reconstruction would lead to a better resulting reconstructed image. (L1 reconstruction is the solution of equation (3) above with q=1 in $J_q(z)$).

The approach described above can be extended to exploit spatial-temporal coherence and to provide the facility for 4D (dynamic 3D) PAT, particularly in view of the use of compressed sensing as described above to reduce image acquisition time. Such dynamic PAT represents a powerful tool for investigating physiological processes such as heart rate beating and breathing (for example). In one embodiment, this 4D PAT processes the data in its spatial Fourier representation as a function of time ($\mathbf{k}$-$t$-space), in which the dynamic images of natural objects exhibit significant correlations - hence it is feasible to acquire only a reduced amount of data, while still obtaining improved temporal resolution. A training set is obtained from which signal correlations can be learned, thereby allowing missing data to be recovered using all available information in a consistent and integral manner [28]. In one embodiment, dictionary learning, which characterises typical images via local patches, can be combined with $\mathbf{k}$-space trajectory schemes to give further improvements [14].

We can describe the dynamic PAT data in terms of two scales of time $y(\mathbf{m}, t; \tau)$, where $\tau$ represents a scale of seconds representing image motion and kinetics. By taking a Fourier Transform in the detector plane coordinates $\mathbf{m} \rightarrow \mathbf{k}_{\parallel}$, and in the acoustic time variable $t \rightarrow \omega$, the $\mathbf{k}$-space representation of the data is obtained, [17], and dynamic imaging is then the acquisition of data in the $\mathbf{k}$-$\tau$ space. The $\mathbf{k}$-$t$ SENSE scheme [28], or other appropriate $\mathbf{k}$-space trajectory scheme, can be adapted for PAT data, having regard to the fact that only two dimensions of $\mathbf{k}$-space are under the control of the experimenter (i.e. $\mathbf{k}_{||}$, corresponding to the two-dimensions of the pattern applied by the micromirror array 170), and also that the micromirror array 170 only supports non-negative patterns. In one embodiment, the data is processed using Markov time series methods, such as Kalman filters, which treat the spatial components of the image as coefficients of random variables which evolve over time, and therefore can be used continuously on real-time data to track changes in specific spatial-temporal components [22].

In one example, an explicit kinetic model is used to derive images of rate constants for the uptake of molecules of interest, such as for kinematic studies of molecular and genomic imaging. Typically these models involve identifying two or more tissue compartments and defining a set of ordinary differential equations linking them. These models can be utilised at both the voxel level and a segmented organ level. One possibility is to apply (non-linear) estimation of the rate constant parameters to reconstructed 4D PAT images. Alternatively, it may be possible to use a direct method, in which the rate constants are recovered directly from the data without an explicit image reconstruction step (this has been shown to be more robust in other kinetic modalities [29]).

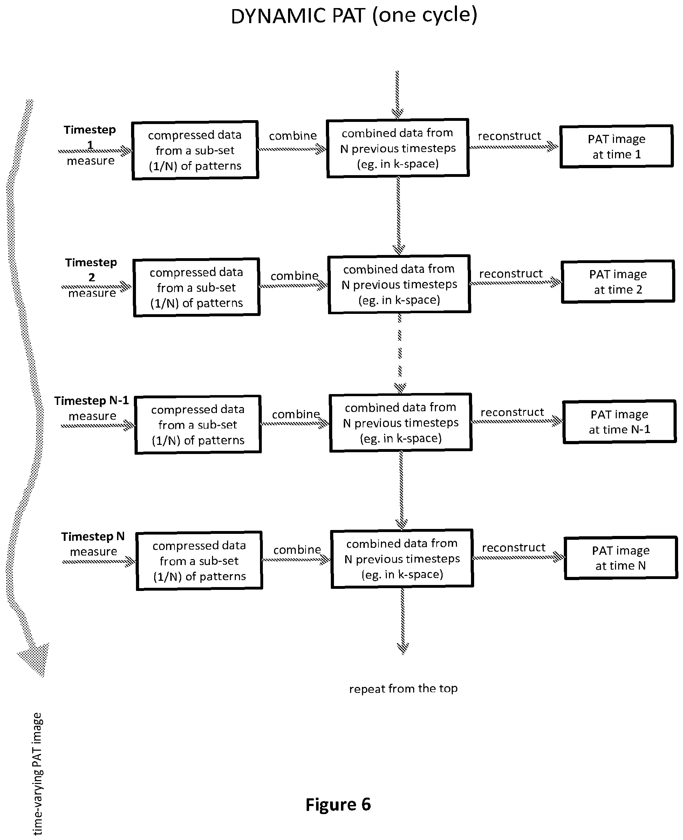

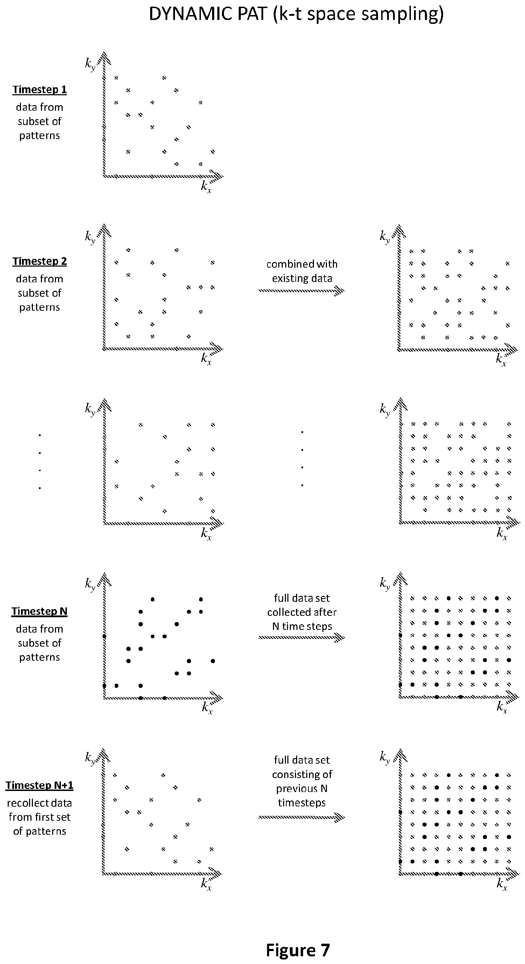

Figures 6 and 7 illustrate in schematic form the above approach to reconstructing dynamic (4D) PAT images according to an example. We assume that the total number of patterns for the sensing is PT and the time for sensing one pattern (i.e. for producing a single output value) is t1. The overall set of PT patterns is divided into N subsets, where each subset contains PT/N patterns, and hence represents a duration of t2=PT*t1/N. The duration t2 in effect represents the frame rate of the dynamic PAT data, in that data within a time period t2 is accumulated to form a single image. Therefore t2 can be regarded as corresponding to $\tau$ as discussed above, which typically represents a scale of seconds for image motion and kinetics.

As shown in Figure 6, for the first timestep (at t=t2), compressed data is collected from a subset of 1/N of the total number (PT) of patterns. This data is then combined with the data in k-space from the previous N timesteps (t=-(N-1)*t2 through to t=0) (to the extent such earlier data is available) in order to generate (reconstruct) the PAT image at time t=t2. For the second timestep (at t=2*t2), compressed data is then collected from the next subset of 1/N of the total number (PT) of patterns. This data is combined with the data from the previous N timesteps (t=-(N-2)*t2 through to t=t2) in order to reconstruct the PAT image at time t=2*t2. This process then repeats continuously, whereby the PAT image at time t=n*t2 is generated by combining the received compressed data for the timestep n*t2 with the data from the previous N timesteps.

Figure 7 illustrates certain aspects of the processing of Figure 6 in more detail in accordance with one example. In particular, Figure 7 depicts each subset of the patterns as a (different) set of locations in k-t space. Thus at timestep 1 (t=t2), the acquired data corresponds to the pattern of locations in k-t space as illustrated (where each location represents a single pattern, and hence a single measurement). For Figure 7, it is assumed that no data is available prior to this first timestep, hence the image reconstruction for timestep 1 is based just on this received data (without combining with any earlier data).

At timestep 2 in Figure 7 (t=2*t2), compressed data is received corresponding to the patterns representing a different subset of locations in k-t space. This data for timestep 2 is combined with the data from timestep 1 to provide a more complete (accurate) reconstruction of the PAT image. This process then repeats until at timestep N, all the patterns, and hence all the locations in k-t space have been sampled. Consequently, for the next timestep, t=(N+1)***t**, we start again with the same subset of sampling patterns as used for timestep 1 (t=t2), and the received data for this subset overwrites the data from timestep 1 at the corresponding locations in k-t space. This process repeats, such that for each new timestep, the data from N timesteps earlier is replaced in k-t space with the newly acquired data for that timestep. The output of this processing is therefore dynamic PAT data, in which the image data is updated (modified) every timestep - i.e. at a frequency of 1/t2.

In one embodiment, wherein the sensitivity patterns are taken from a set of linear combinations of Fourier patterns, such that over a dynamic sequence a full sampling of Fourier space (**k**-space) in coordinates of the acoustically sensitive surface is achieved or can be reconstructed. For example, the Fourier sampling may be reconstructed or the standard k-t-tau algorithms adapted to work with the compressed Fourier sampling.

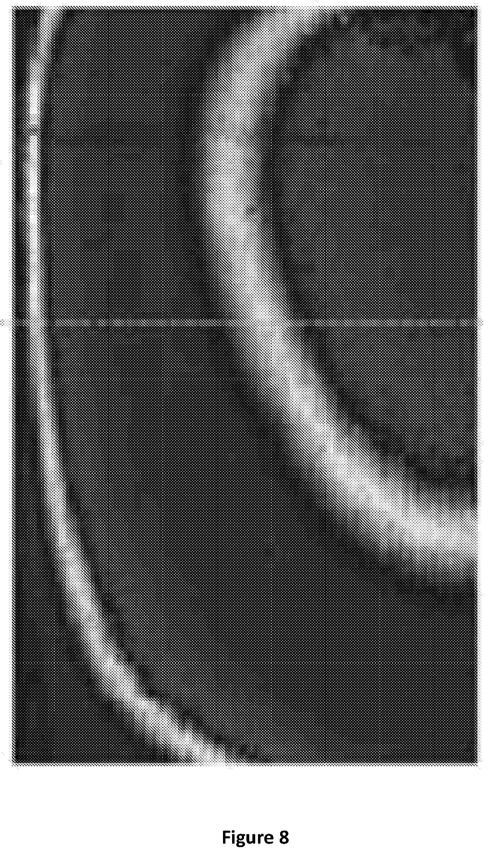

Returning to Figure 2, the FP interferometer detector 20 may exhibit sensitivity variations across its surface due to variations in optical thickness. This means that for any given wavelength of the interrogation beam 132, only a limited portion of the detector is sensitive - for example, Figure 8 illustrates a typical pattern in which there are a few contours of uniform sensitivity, as illustrated by the bright regions in the Figure, while the dark regions between these contours are acoustically insensitive.

In order to interrogate the FP interferometer detector 20, a pre-tuning procedure is performed before acquiring the photoacoustic image. In one example, this procedure involves illuminating the FP interferometer detector 20 pixel by pixel. At each pixel, the reflected light intensity is recorded as a function of wavelength, termed the wavelength interferometer transfer function (ITF). The wavelength corresponding to the maximum value of the derivative of the ITF is called the optimum bias wavelength and is the wavelength that yields the highest sensitivity for that pixel. Thus the pre-tuning procedure yields a pixel-by-pixel map of the optimum bias wavelengths.

Once the optimum bias wavelength map has been acquired, the interrogation laser 10 is set to a specific bias wavelength and then, using the micromirror array 170, only those regions that provide high sensitivity at that wavelength are illuminated - in the context of compressed sensing, the sensitivity pattern would only be applied to this region - and the photoacoustic signal is recorded. The wavelength of the interrogation laser 10 is then adjusted to make a different part of the sensor sensitive and the photoacoustic signal is recorded again. This process is repeated for a range of wavelengths until the entire sensing region of the FP interferometer detector has been mapped - in essence varying the wavelength allows the sensitivity contours to be shifted (scanned) across the FP interferometer detector 20. The advantage of selectively illuminating the sensitive regions in this way is that it avoids detection of the high un-modulated dc optical intensity reflected from the insensitive regions which would otherwise saturate the detector and increase noise significantly.

Note that the sensitivity pattern of the FP interferometer detector 20 is generally dependent on the properties of the FP interferometer detector 20. The pre-tuning procedure may be avoided (in the sense that the optimum bias wavelength is the same across the detector) if the FP interferometer detector 20 has a very consistent thickness across all the acoustically sensitive surface, or if the finesse of the FP interferometer detector 20 is reduced (although the latter approach will lead to a photoacoustic image likewise having lower sensitivity or discrimination between different signal levels).

Although the present invention has been described in the context of particular embodiments, the skilled person will be aware of a range of other possible implementations. For example, rather than using a Fabry-Perot interferometer to perform the sensing of the acoustic field, as shown in Figure 2, various other techniques and apparatus for encoding the acoustic field on to an optical signal exist. These include the use of a multilayer optical hydrophone, such as described in [35: Klann and Koch], and the use of a glass prism covered with a water layer, such as described in [36: Köstli et al.], both of which involve the acoustic field modulating the reflected power of an incident laser beam. The device of Klann and Koch is conceptually similar to an FP interferometer detector but uses hard dielectric materials. As a result, the spatial variations in sensitivity are less than with an FP sensor, since the hard dielectric materials can be deposited with greater precision, but the device is generally not as sensitive as an FP sensor. The device of Kostli is a non-interferometric intensity-based sensor which avoids the use of an expensive narrow linewidth tunable interrogation laser. This is generally able to provide uniform spatial sensitivity, but is typically less sensitive than an FP sensor. Yakovlev [37] provides another non-interferometric intensity-based sensor in which a plasmonic metamaterial or metal is deposited onto a transparent substrate. This has the same advantages as the device of Kostli, but has higher sensitivity. A slightly different approach, in which the acoustic field impinging on a sensing surface (pellicle) is used to modulate the phase of a reflected laser beam, is disclosed in [38: Hamilton et al.]. This is a form of interferometric sensor, but unlike an FP sensor it provides an absolute measurement of displacement.

Devices which are less prone to sensitivity variations across the acoustically sensitive surface are beneficial, providing they can provide sufficient sensitivity, since they potentially support faster image acquisition by allowing the whole surface (or at least more of the surface) of the device to be operational simultaneously.

The compressed sensing pattern (as shown in Figure 3) is then applied to the incident laser beam (or potentially to the reflected laser beam) so as to produce a spatial weighting of the acoustic signal across the sensor surface in accordance with the applied sensing pattern. This spatially weighted version of the acoustic signal is then passed to a detector to obtain a single output value. A sequence of such (compressed) measurements can then be reconstructed into a single image, and then potentially into a sequence of images, for dynamic PAT, as described above.

A number of different devices are available to apply a sequence of sensitivity patterns to the interrogation beam instead of the micromirror array 170 - such as a spatial light modulator, a rotating wheel or polygon bearing a set of masks or mirrors across the different faces, a sliding linear strip of such masks or mirrors (or any other suitable configuration), or other types of device based on adaptive optics. A particular benefit of using a device such as a micromirror array or a spatial light modulator to apply the sensitivity patterns (rather than a rotating wheel, for example, which is provided with a fixed set and order of mask patterns) is that such a device can apply a configurable sequence of sensitivity patterns. In other words, for a rotating wheel (for example), the sequence of sensitivity patterns is, in effect, hard-coded into the sequence of mask or mirrors arranged around the wheel itself. However, for a configurable device, such as a spatial light modulator or micromirror array, the sequence of sensitivity patterns is readily programmable (i.e. customisable or configurable), which means for example that the system can be readily altered to accommodate different sets or ordering of sensitivity patterns.

In some embodiments, a single sensitivity pattern is applied for each pulse of excitation radiation. In other embodiments, a sequence of two or more sensitivity patterns is applied for each pulse of excitation radiation. In other words, a given pulse of excitation radiation results in a time series representing the acoustic signal generated by the acoustically-sensitive surface in response to the acoustic wave caused by the excitation pulse, and this time series may correspond to more than one sensitivity pattern. The sensitivity pattern which is applied to the interrogation beam may then be changed (one or more times) partway through the acoustic signal (and corresponding time series) arising from the given pulse of excitation radiation. This approach could be implemented, for example, using an array of high speed optical modulators (to provide the rapid control and switching between the different applied sensitivity patterns).

Another potential modification of the apparatus of Figure 2 is that the micromirror array 170 (or other mechanism for applying a sensing pattern) is located downstream of the sensor head 200 in the overall optical path. For example, the micromirror array may be located, between the beam splitter 122 and the objective lens 162, or between the objective lens 162 and the photodiode 150 (with an appropriate change in the remaining layout to accommodate this change in configuration).

Furthermore, in relation to the dynamic imaging approach shown in Figures 6 and 7, PT (the number of patterns) does not necessarily need to fill out the k-t space, whereby the number of timesteps to be combined into a single PAT image (frame) is less than N In other words, the dynamic imaging is used in addition to the compressed sensing, so that the recovery of each frame of the dynamic image benefits from both compressed sensing and spatially-temporal correlation exploited through k-t space schemes.

Additional implementation examples can be found in [39].

The above embodiments involving various data processing, such as image reconstruction, which may be performed by specialised hardware, by general purpose hardware running appropriate computer code, or by some combination of the two. For example, the general purpose hardware may comprise a personal computer, a computer workstation, etc. The computer code may comprise computer program instructions that are executed by one or more processors to perform the desired operations. The one or more processors may be located in or integrated into special purpose apparatus, such as a PAT acquisition system. The one or more processors may comprise digital signal processors, graphics processing units, central processing units, or any other suitable device. The computer program code is generally stored in a non-transitory medium such as an optical disk, flash memory (ROM), or hard drive, and then loaded into random access memory (RAM) prior to access by the one or more processors for execution.

In conclusion, the skilled person will be aware of various modifications that can be made to the above embodiments to reflect the particular circumstances of any given implementation. Moreover, the skilled person will be aware that features from different embodiments can be combined as appropriate in any given implementation. Accordingly, the scope of the present invention is defined by the appended claims.

## References

1. T. Allen, A. Hall, A.P. Dhillon, and P. Beard. J. Biomed. Opt., 17(6), 2012.

2. P. Beard. Interface Focus, 1(4):602-631, 2011.

3. J. M. Bioucas-Dias and M. A. T. Figueiredo. IEEE Trans. Im. Proc., 16(12):2992-3004, 2007.

4. A. M. Brookstein, D. L. Donoho, and M. Elad. SIAM Review, 51(1):34-81, May 2009.

5. E. Candes, L. Demanet, D. Donoho, and L. Ying. Multiscale Model. Sim., 5(3):861-899, 2006.

6. E. J. Candes and L. Demanet. Comm. Pure and Applied Math., 58(11):1472-1528, 2005.

7. E. J. Candes and T. Tao. IEEE Trans. Inf. Theory, 52(12):5406-5425, 2006.

8. E. J. Candes, M. B. Wakin, and S. P. Boyd. J. Fourier Anal. Appl., 14(5-6):877-905, 2008.

9. B. T. Cox, S. Kara, S. R. Arridge, and P. C. Beard. J. Acoust. Soc. Am., 121(6):3453-64, 2007.

10. M. N. Do and M. Vetterli. IEEE Trans. Im. Proc., 14:2091-2106, 2005.

11. D. L. Donoho, A. Maleki, and A. Montanari. PNAS, 106(45):18914-18919, Nov. 2009.

12. M. Duarte et.al., Signal Processing Magazine, IEEE, 25(2):83 -91, march 2008.

13. Z. Guo, C. Li, L. Song, and L. V. Wang. J. Biomed. Opt., 15(2):021311, 2011.

14. H. Jung, J. C. Ye, and E. Y. Kim. Phys. Med. Biol., 52:3201-3226, 2007.

15. J. Laufer et.al., J. Biomed. Opt., 17(6), 2012.

16. J. Laufer et.al., J. Biomed. Opt., 17(5), 2012.

17. K. P. Koestli, M. Frenz, H. Bebie, and H. P. Weber. Phys. Med. Biol., 46(7):1863-72, 2001.

18. K. Kreutz-Delgado et.al., Neural Comp., 15:349-396, 2003.

19. R. A. Kruger, R. B. Lam, D. R. Reinecke, S. P. Del Rio, and R. P. Doyle. Med. Phys., 37(11):6096, 2010.

20. G. Kutyniok, M. Shahram, and X. Zhuang. arXiv.org, math.NA, June 2011.

21. M.Lustig, D. Donoho, and J. Pauly. Magn Reson Med, 58:1182-1195, 2007.

22. S. Prince et.al., Phys. Med. Biol., 48(11):1491-1504, 2003.

23. J. Provost and F. Lesage. IEEE Trans. Med. Im., 28(4):585-94, 2009.

24. M. Sun, N. Feng, Y. Shen, X. Shen, L. Ma, J. Li, and Z. Wu. Optics Exp., 19(16):14801?-14806, 2011.

25. J. Tang, B. E. Nett, and G. H. Chen. Med. Phys., 54:5781-5804, 2009.

26. B. E. Treeby and B. T. Cox. J. Biomed. Opt., 15(2):021314, 2010.

27. J. A. Tropp and A. C. Gilbert. IEEE Trans. Inf. Theory, 53(12):4655-4666, 2007.

28. J. Tsao, P. Boesiger, and K. P. Pruessmann. Magn Reson Med, 50(5):1031-1042, Nov 2003.

29. G. Wang, and J. Qi. IEEE Trans. Med. Im., 28(11):1717-1726, 2009.

30. L. Wang. Photoacoustic Imaging and Spectroscopy. CRC Press, 2009.

31. X. Wang, Y. Pang, G. Ku, X. Xie, G. Stocia, and L. V. Wang. Nature Biotechnology, 21(7):803-806, 2003.

32. W. Yin, S. Osher, D. Goldfarb, and J. Darbon. SIAM Journal on Imaging Sciences, 1(1):143-168, 2008.

33. E. Z. Zhang, J. G. Laufer, R. B. Pedley, and P. C. Beard. Phys. Med. Biol., 54(4):1035-46, 2009.

34. "Review: Biomedical Photoacoustic Imaging" by P.C. Beard, Interface Focus, 2001, 1, 602-631, first published online 21 June 2011.

35. M. Klann and C Koch. IEEE Trans. on Ultrasonics, Ferroelectrics and Frequency Control, v52(9),1546-1554, 2005.

36. K. P. Koestli et al. Applied Optics 40(22): 3800-3809 2001.

37. V. V. Yakovlev et al. Advanced Materials, Wiley online library, DOI:10.1002/adma.201300314, 1-6 2013.

38. J.D. Hamilton et al, IEEE Trans. on Ultrasonics, Ferroelectrics and Frequency Control, v47(1),160-169, 2000.

39. Huynh et al, Proc. Of SPIE: Photons plus Ultrasound: Imaging and Sensing, v8943, 894327-1, March 2014.

**Claims**

1. Apparatus for performing photoacoustic tomography with respect to a sample (12) that receives a pulse of excitation electromagnetic radiation and generates an acoustic field in response to said pulse, said apparatus comprising:

   an acoustically sensitive surface (20), wherein the acoustic field generated in response to said pulse is incident upon said acoustically sensitive surface to form a signal;
   a source for directing an interrogation beam (131) of electromagnetic radiation onto said acoustically sensitive surface so as to be modulated by the signal;
   means (170) for applying a sensitivity pattern to the interrogation beam; and
   a read-out system (150, 155) for receiving the interrogation beam from the acoustically sensitive surface and for determining a value representing a spatial integral of the signal across the acoustically sensitive surface, wherein said spatial integral is weighted by the applied sensitivity pattern;
   wherein the apparatus is configured to apply a sequence of sensitivity patterns to the interrogation beam and to determine a respective sequence of values for said weighted spatial integral for generating a photoacoustic image.

2. The apparatus of claim 1, wherein the interrogation beam comprises an optical, infrared or microwave laser.

3. The apparatus of claim 1 or 2, further comprising a multilayer optical device such as a hydrophone, a glass prism covered with a layer of water, a pellicle, or a plasmonic metamaterial or metal deposited onto a transparent substrate, wherein the acoustically sensitive surface is part of the multilayer optical device.

4. The apparatus of claim 1 or 2, further comprising a thin film Fabry-Perot sensor head having a thickness which is subject to acoustically induced modulation in response to the incident acoustic field, wherein the acoustically sensitive surface is part of the thin film Fabry-Perot sensor head.

5. The apparatus of claim 1 or 2, wherein the acoustically sensitivity surface has a substantially uniform sensitivity across the surface.

6. The apparatus of any preceding claim, wherein the means for applying the sensitivity pattern is able to apply a programmable sequence of sensitivity patterns, and optionally wherein the means for applying a sensitivity pattern to the interrogation beam comprises a micromirror array that reflects the interrogation beam in accordance with said sensitivity pattern.

7. The apparatus of any of claims 1 to 5, wherein the means for applying the sensitivity pattern comprises a moving film or wheel with masks, a micromirror array, or a spatial light modulator, and optionally wherein the means for applying a sensitivity pattern to the interrogation beam comprises a micromirror array that reflects the interrogation beam in accordance with said sensitivity pattern.

8. The apparatus of any preceding claim, wherein the read-out system comprises a photodetector for receiving the interrogation beam from the acoustically sensitive surface.

9. The apparatus of any preceding claim, wherein for each applied sensitivity pattern, the read-out system is configured to generate a data set comprising a time series of the value for said spatial integral, and optionally wherein the apparatus is configured to apply: (i) a single sensitivity pattern, or (ii) a sequence of multiple sensitivity patterns, during detection of the acoustic field resulting from one pulse of excitation radiation; and optionally wherein the sensitivity patterns are incoherent to a curvelet or shearlet basis.

10. The apparatus of claim 8, wherein the apparatus is configured to:

slice the time series for each applied sensitivity pattern in said sequence, thereby producing for each time slice, a set of data values, each data value corresponding to an applied sensitivity pattern;
reconstruct, for a given time slice, across all the applied sensitivity patterns in said sequence, an image of the incident acoustic field for that time slice; and
reconstruct, from the reconstructed images of said time slices, a three-dimensional initial pressure distribution.

11. The apparatus of any preceding claim, wherein the apparatus is configured to apply different sensitivity patterns at successive time samples, $\tau_i$, of a dynamic sequence; and optionally wherein the apparatus is configured to use a Markov time-series approach for reconstruction to generate a photoacoustic image, time-step by time-step; and optionally wherein the apparatus is configured to use dictionary learning to reduce the number of required sensitivity patterns, with a commensurate increase in the dynamic imaging rate.

12. The apparatus of claim 11, wherein the sensitivity patterns are taken from a set of linear combinations of Fourier patterns, such that over a dynamic sequence a full sampling of Fourier space (k-space) in coordinates of the acoustically sensitive surface is achieved or can be reconstructed, and optionally wherein the apparatus is further configured to reconstruct, from measurements in k-t-$\tau$ space, a 4D image in $(x,y,z,\tau)$ space.

13. The apparatus of any preceding claim, wherein the apparatus is configured to:

perform a pre-tuning procedure before acquiring the photoacoustic image by recording the reflected light intensity as a function of wavelength across the acoustically-sensitive surface;
and optionally wherein the pre-tuning procedure further comprises selecting, for different locations of the acoustically-sensitive surface, the wavelength corresponding to the maximum value of the derivative of the recorded reflected light intensity as a function of wavelength, wherein the selected wavelength represents the optimum bias wavelength, thereby generating a set of optimum bias wavelengths corresponding to said different locations of the acoustically-sensitive surface;
and optionally wherein the apparatus is configured to repeat, for each optimum bias wavelength in said set of optimum bias wavelengths, the application of a sequence of sensitivity patterns to the interrogation beam for determining a respective sequence of values for said weighted spatial integral, wherein for each optimum bias wavelength the apparatus is further configured to apply a window to restrict the spatial integral to the one or more locations of the acoustically-sensitive surface corresponding to that optimum bias wavelength.

14. A method for performing photoacoustic tomography with respect to a sample that receives a pulse of excitation electromagnetic radiation and generates an acoustic field in response to said pulse, said method comprising:

forming a signal using an acoustically sensitive surface, wherein the signal represents the acoustic field which is generated in response to said pulse, as incident upon said acoustically sensitive surface;
directing an interrogation beam of electromagnetic radiation onto said acoustically sensitive surface so as to be modulated by the signal;
applying a sensitivity pattern to the interrogation beam;
receiving at a read-out system the interrogation beam from the acoustically sensitive surface and determining a value representing a spatial integral of the signal across the acoustically sensitive surface, wherein said spatial integral is weighted by the applied sensitivity pattern;
applying a sequence of sensitivity patterns to the interrogation beam to determine a respective sequence of values for said weighted spatial integral for generating a photoacoustic image.

15. The method of claim 14, further comprising performing an image reconstruction process on said sequence of values for generating said photoacoustic image.

**Patentansprüche**

1. Vorrichtung zur Durchführung einer photoakustischen Tomografie in Bezug auf eine Probe (12), die einen Puls elektromagnetischer Anregungsstrahlung empfängt und ein akustisches Feld in Antwort auf den Puls erzeugt, die Vorrichtung aufweisend:

   eine akustisch empfindliche Fläche (20), wobei das akustische Feld, das in Antwort auf den Puls erzeugt wird, auf die akustisch empfindliche Fläche trifft, um ein Signal zu bilden;
   eine Quelle zum Richten eines Abfragestrahls (131) elektromagnetischer Strahlung auf die akustisch empfindliche Fläche, um durch das Signal moduliert zu werden;
   Mittel (170) zum Anwenden eines Empfindlichkeitsmusters auf den Abfragestrahl; und
   ein Auslesesystem (150, 155) zum Empfangen des Abfragestrahls von der akustisch empfindlichen Fläche und zum Ermitteln eines Werts, der ein räumliches Integral des Signals über die akustisch empfindliche Fläche darstellt, wobei das räumliche Integral durch das angewendete Empfindlichkeitsmuster gewichtet ist;
   wobei die Vorrichtung konfiguriert ist, eine Sequenz von Empfindlichkeitsmustern auf den Abfragestrahl anzuwenden und eine jeweilige Sequenz von Werten für das gewichtete räumliche Integral zum Erzeugen eines photoakustischen Bilds zu ermitteln.

2. Vorrichtung nach Anspruch 1, wobei der Abfragestrahl einen optischen, infraroten oder Mikrowellenlaser aufweist.

3. Vorrichtung nach Anspruch 1 oder 2, ferner aufweisend ein mehrschichtiges optisches Gerät, wie ein Hydrophon, ein Glasprisma, das mit einer Schicht aus Wasser bedeckt ist, ein Pellikel oder ein plasmonisches Metamaterial oder Metall, das auf einem durchsichtigen Substrat abgeschieden ist, wobei die akustisch empfindliche Fläche Teil des mehrschichtigen optischen Geräts ist.

4. Vorrichtung nach Anspruch 1 oder 2, ferner aufweisend einen Dünnfilm-Fabry-Perot-Sensorkopf mit einer Dicke, die akustisch induzierter Modulation in Antwort auf das einfallende akustische Feld unterzogen wird, wobei die akustisch empfindliche Fläche Teil des Dünnfilm-Fabry-Perot-Sensorkopfs ist.

5. Vorrichtung nach Anspruch 1 oder 2, wobei die akustische Empfindlichkeitsfläche eine im Wesentlichen einheitliche Empfindlichkeit über die Fläche hat.

6. Vorrichtung nach einem der vorangehenden Ansprüche, wobei die Mittel zum Anwenden des Empfindlichkeitsmusters fähig ist, eine programmierbare Sequenz von Empfindlichkeitsmustern anzuwenden und optional wobei die Mittel zum Anwenden eines Empfindlichkeitsmusters an dem Abfragestrahl ein Mikrospiegelfeld aufweist, das den Abfragestrahl in Übereinstimmung mit dem Empfindlichkeitsmuster reflektiert.

7. Vorrichtung nach einem der Ansprüche 1 bis 5, wobei das Mittel zum Anwenden des Empfindlichkeitsmusters einen beweglichen Film oder ein Rad mit Masken, ein Mikrospiegelfeld oder einen räumlichen Lichtmodulator aufweist und optional wobei das Mittel zum Anwenden eines Empfindlichkeitsmusters an dem Abfragestrahls ein Mikrospiegelfeld aufweist, das den Abfragestrahl in Übereinstimmung mit dem Empfindlichkeitsmuster reflektiert.

8. Vorrichtung nach einem der vorangehenden Ansprüche, wobei das Auslesesystem einen Photodetektor zum Empfangen des Abfragestrahls von der akustisch empfindlichen Fläche aufweist.

9. Vorrichtung nach einem der vorangehenden Ansprüche, wobei für jedes angewendete Empfindlichkeitsmuster das Auslesesystem konfiguriert ist, einen Datensatz zu erzeugen, der eine Zeitserie des Werts für das räumliche Integral aufweist und optional wobei die Vorrichtung konfiguriert ist anzuwenden: (i) ein einzelnes Empfindlichkeitsmuster oder (ii) eine Sequenz mehrerer Empfindlichkeitsmuster, während Erfassung des akustischen Felds, das aus einem Puls von Anregungsstrahlung resultiert; und optional wobei die Empfindlichkeitsmuster zu einer Curvelet- oder Shearlet-Basis inkohärent sind.

10. Vorrichtung nach Anspruch 8, wobei die Vorrichtung konfiguriert ist zum:

    Teilen der Zeitserien für jedes angewendete Empfindlichkeitsmuster in der Sequenz, wodurch für jeden Zeitanteil ein Satz von Datenwerten erzeugt wird, wobei jeder Datenwert einem angewendeten Empfindlichkeitsmuster entspricht;
    Rekonstruieren, für einen gegebenen Zeitanteil, über alle der angewendeten Empfindlichkeitsmuster in der Sequenz, eines Bilds des eintreffenden akustischen Felds für diesen Zeitanteil; und
Rekonstruieren, aus den rekonstruierten Bildern der Zeitanteile, einer dreidimensionalen Anfangsdruckverteilung.

11. Vorrichtung nach einem der vorangehenden Ansprüche, wobei die Vorrichtung konfiguriert ist, verschiedene Empfindlichkeitsmuster bei aufeinanderfolgenden Zeitabtastungen, $\tau_i$, einer dynamischen Sequenz anzuwenden; und wobei die Vorrichtung optional konfiguriert ist, eine Markov-Zeitkettenmethode zur Rekonstruktion zu verwenden, um Zeitschritt für Zeitschritt ein photoakustisches Bild zu erzeugen; und wobei die Vorrichtung optional konfiguriert ist, Lexikonlernen zu verwenden, um die Zahl benötigter Empfindlichkeitsmuster zu verringern, mit einer proportionalen Zunahme der dynamischen Abbildungsrate.

12. Vorrichtung nach Anspruch 11, wobei die Empfindlichkeitsmuster aus einem Satz linearer Kombinationen von Fourier-Mustern genommen werden, sodass über eine dynamische Sequenz eine vollständige Abtastung von Fourier-Raum (**k**-Raum) in Koordinaten der akustisch empfindlichen Fläche erzielt wird oder rekonstruiert werden kann, und wobei optional die Vorrichtung ferner konfiguriert ist, aus Messungen in k-t-$\tau$ Raum ein 4D-Bild im (x,y,z,$\tau$) Raum zu rekonstruieren.

13. Vorrichtung nach einem der vorangehenden Ansprüche, wobei die Vorrichtung konfiguriert ist zum:

Durchführen einer Vorabstimmungsprozedur vor Erlangen des photoakustischen Bilds durch Aufzeichnen der reflektierten Lichtintensität als eine Funktion von Wellenlänge über die akustisch-empfindliche Fläche; und wobei die Voreinstellungsprozedur optional ferner Auswählen, für verschiedene Stellen der akustisch-empfindlichen Fläche, der Wellenlänge entsprechend dem maximalen Wert der Ableitung der aufgezeichneten Lichtintensität als eine Funktion von Wellenlänge aufweist, wobei die ausgewählte Wellenlänge die optimale Vorspannwellenlänge darstellt, wodurch ein Satz optimaler Vorspannwellenlängen entsprechend der verschiedenen Stellen der akustisch-empfindlichen Fläche erzeugt wird; und wobei die Vorrichtung optional konfiguriert ist, für jede optimale Vorspannwellenlänge im Satz optimaler Vorspannwellenlängen die Anwendung einer Sequenz von Empfindlichkeitsmustern am Abfragestrahl zum Ermitteln einer jeweiligen Sequenz von Werten für das gewichtete räumliche Integral zu wiederholen, wobei für jede optimale Vorspannwellenlänge die Vorrichtung ferner konfiguriert ist, ein Fenster anzuwenden, um das räumliche Integral auf die eine oder mehr Stellen der akustisch-empfindlichen Fläche entsprechend dieser optimalen Vorspannwellenlänge einzuschränken.

14. Verfahren zur Durchführung einer photoakustischen Tomografie in Bezug auf eine Probe, die einen Puls elektromagnetischer Anregungsstrahlung empfängt und ein akustisches Feld in Antwort auf den Puls erzeugt, das Verfahren aufweisend:

Bilden eines Signals unter Verwendung einer akustisch empfindlichen Fläche, wobei das Signal das akustische Feld, das in Antwort auf den Puls erzeugt ist, als auf die akustisch empfindliche Fläche treffend darstellt; Leiten eines Abfragestrahls elektromagnetischer Strahlung auf die akustisch empfindliche Fläche, um durch das Signal moduliert zu werden; Anwenden eines Empfindlichkeitsmusters auf den Abfragestrahl; Empfangen eines Auslesesystems des Abfragestrahls von der akustisch empfindlichen Fläche und Ermitteln eines Werts, der ein räumliches Integral des Signals über die akustisch empfindliche Fläche darstellt, wobei das räumliche Integral durch das angewendete Empfindlichkeitsmuster gewichtet ist; Anwenden einer Sequenz von Empfindlichkeitsmustern auf den Abfragestrahl, um eine jeweilige Sequenz von Werten für das gewichtete räumliche Integral zur Erzeugung eines photoakustischen Bilds zu ermitteln.

15. Verfahren nach Anspruch 14, ferner aufweisend Durchführen eines Bildrekonstruktionsprozesses an der Sequenz von Werten, zum Erzeugen des photoakustischen Bilds.

**Revendications**

1. Appareil destiné à effectuer une tomographie photoacoustique sur un échantillon (12) qui reçoit une impulsion de rayonnement électromagnétique d'excitation et génère un champ acoustique en réponse à ladite impulsion, ledit appareil comprenant :

une surface acoustiquement sensible (20), le champ acoustique généré en réponse à ladite impulsion étant incident sur ladite surface acoustiquement sensible pour former un signal ;

une source destinée à diriger un faisceau d'interrogation (131) de rayonnement électromagnétique sur ladite surface acoustiquement sensible de manière à ce qu'il soit modulé par le signal ;

un moyen (170) pour appliquer un motif de sensibilité au faisceau d'interrogation ; et

un système de lecture (150, 155) destiné à recevoir le faisceau d'interrogation provenant de la surface acoustiquement sensible et à déterminer une valeur représentant une intégrale spatiale du signal à travers la surface acoustiquement sensible, ladite intégrale spatiale étant pondérée par le motif de sensibilité appliqué ;

l'appareil étant configuré pour appliquer une séquence de motifs de sensibilité au faisceau d'interrogation et pour déterminer une séquence respective de valeurs pour ladite intégrale spatiale pondérée afin de générer une image photoacoustique.

2. Appareil de la revendication 1, dans lequel le faisceau d'interrogation comprend un laser optique, infrarouge ou à micro-ondes.

3. Appareil de la revendication 1 ou 2, comprenant en outre un dispositif optique multicouche tel qu'un hydrophone, un prisme en verre recouvert d'une couche d'eau, une pellicule, ou un métamatériau plasmonique ou un métal déposé sur un substrat transparent, la surface acoustiquement sensible faisant partie du dispositif optique multicouche.

4. Appareil de la revendication 1 ou 2, comprenant en outre une tête de détection de Fabry-Pérot en couche mince ayant une épaisseur qui est soumise à une modulation induite acoustiquement en réponse au champ acoustique incident, la surface acoustiquement sensible faisant partie de la tête de détection de Fabry-Pérot en couche mince.

5. Appareil de la revendication 1 ou 2, dans lequel la surface de sensibilité acoustique a une sensibilité sensiblement uniforme d'un bout à l'autre de la surface.

6. Appareil d'une quelconque revendication précédente, dans lequel le moyen pour appliquer le motif de sensibilité peut appliquer une séquence programmable de motifs de sensibilité, et éventuellement dans lequel le moyen pour appliquer un motif de sensibilité au faisceau d'interrogation comprend un réseau de micromiroirs qui réfléchit le faisceau d'interrogation en fonction dudit motif de sensibilité.

7. Appareil de l'une quelconque des revendications 1 à 5, dans lequel le moyen pour appliquer le motif de sensibilité comprend un film mobile ou une roue avec des masques, un réseau de micromiroirs, ou un modulateur spatial de lumière, et éventuellement dans lequel le moyen pour appliquer un motif de sensibilité au faisceau d'interrogation comprend un réseau de micromiroirs qui réfléchit le faisceau d'interrogation en fonction dudit motif de sensibilité.

8. Appareil d'une quelconque revendication précédente, dans lequel le système de lecture comprend un photodétecteur destiné à recevoir le faisceau d'interrogation provenant de la surface acoustiquement sensible.

9. Appareil d'une quelconque revendication précédente dans lequel, pour chaque motif de sensibilité appliqué, le système de lecture est configuré pour générer un ensemble de données comprenant une série temporelle de la valeur pour ladite intégrale spatiale, et l'appareil étant éventuellement configuré pour appliquer : (i) un seul motif de sensibilité, ou (ii) une séquence de multiples motifs de sensibilité, pendant la détection du champ acoustique résultant d'une impulsion de rayonnement d'excitation ; et éventuellement dans lequel les motifs de sensibilité sont incohérents en termes de courbelettes ou d'ondelettes de cisaillement.

10. Appareil de la revendication 8, l'appareil étant configuré pour :

découper la série temporelle pour chaque motif de sensibilité appliqué dans ladite séquence, pour produire ainsi, pour chaque tranche de temps, un ensemble de valeurs de données, chaque valeur de donnée correspondant à un motif de sensibilité appliqué ;

reconstruire, pour une tranche de temps donnée, à travers tous les motifs de sensibilité appliqués dans ladite séquence, une image du champ acoustique incident pour cette tranche de temps ; et

reconstruire, à partir des images reconstruites desdites tranches de temps, une distribution de pression initiale tridimensionnelle.

11. Appareil d'une quelconque revendication précédente, l'appareil étant configuré pour appliquer différents motifs de sensibilité à des échantillons de temps successifs, $\tau_i$, d'une séquence dynamique ; et l'appareil étant éventuellement configuré pour utiliser une approche par les séries temporelles de Markov pour la reconstruction pour générer une image photoacoustique, étape temporelle par étape temporelle ; et l'appareil étant éventuellement configuré pour utiliser un apprentissage de dictionnaire pour réduire le nombre de motifs de sensibilité nécessaires, avec une augmentation proportionnée de la fréquence d'imagerie dynamique.

12. Appareil de la revendication 11, dans lequel les motifs de sensibilité sont pris dans un ensemble de combinaisons linéaires de motifs de Fourier, de telle sorte que, sur une séquence dynamique, un échantillonnage complet de l'espace de Fourier (espace k) en coordonnées de la surface acoustiquement sensible est réalisé ou peut être reconstruit, et l'appareil étant éventuellement aussi configuré pour reconstruire, à partir de mesures dans l'espace k-t-$\tau$, une image 4D dans l'espace (x,y,z,$\tau$).

13. Appareil d'une quelconque revendication précédente, l'appareil étant configuré pour :

effectuer une procédure de préréglage avant d'acquérir l'image photoacoustique en enregistrant l'intensité lumineuse réfléchie en fonction de la longueur d'onde à travers la surface acoustiquement sensible ;
et éventuellement dans lequel la procédure de préréglage comprend en outre la sélection, pour différents emplacements de la surface acoustiquement sensible, de la longueur d'onde correspondant à la valeur maximale de la dérivée de l'intensité lumineuse réfléchie enregistrée en fonction de la longueur d'onde, la longueur d'onde sélectionnée représentant la longueur d'onde de polarisation optimale, pour générer ainsi un ensemble de longueurs d'onde de polarisation optimales correspondant auxdits différents emplacements de la surface acoustiquement sensible ;
et l'appareil étant éventuellement configuré pour répéter, pour chaque longueur d'onde de polarisation optimale dans ledit ensemble de longueurs d'onde de polarisation optimales, l'application d'une séquence de motifs de sensibilité au faisceau d'interrogation pour déterminer une séquence respective de valeurs pour ladite intégrale spatiale pondérée, dans lequel, pour chaque longueur d'onde de polarisation optimale, l'appareil est également configuré pour appliquer une fenêtre pour limiter l'intégrale spatiale à l'emplacement ou aux emplacements de la surface acoustiquement sensible correspondant à cette longueur d'onde de polarisation optimale.

14. Procédé destiné à effectuer une tomographie photoacoustique sur un échantillon qui reçoit une impulsion de rayonnement électromagnétique d'excitation et génère un champ acoustique en réponse à ladite impulsion, ledit procédé comprenant les étapes suivantes :

former un signal en utilisant une surface acoustiquement sensible, le signal représentant le champ acoustique qui est généré en réponse à ladite impulsion, tel qu'incident sur ladite surface acoustiquement sensible ;
diriger un faisceau d'interrogation de rayonnement électromagnétique sur ladite surface acoustiquement sensible de manière à ce qu'il soit modulé par le signal ;
appliquer un motif de sensibilité au faisceau d'interrogation ;
recevoir, au niveau d'un système de lecture, le faisceau d'interrogation provenant de la surface acoustiquement sensible et déterminer une valeur représentant une intégrale spatiale du signal à travers la surface acoustiquement sensible, ladite intégrale spatiale étant pondérée par le motif de sensibilité appliqué ;
appliquer une séquence de motifs de sensibilité au faisceau d'interrogation pour déterminer une séquence respective de valeurs pour ladite intégrale spatiale pondérée afin de générer une image photoacoustique.

15. Procédé de la revendication 14, comprenant en outre la réalisation d'un processus de reconstruction d'image sur ladite séquence de valeurs pour générer ladite image photoacoustique.

**Figure 1**

**Figure 2**

**Figure 3**

Compressed Sensing Data Set (S(t)) 410

Data Set Sliced by time 420

Derive Image Sequence by time (y(m,t)) 430

Reconstruct initial pressure distribution ($p_0$) 440

B

Obtain 3-D PAT image 450

**Figure 4**

B) Full data reconstruction

A) Initial pressure slice

C) Reconstruction from 20% compressed data

Figure 5

# DYNAMIC PAT (one cycle)

**Timestep 1**
measure → compressed data from a sub-set (1/N) of patterns → combine → combined data from N previous timesteps (eg. in k-space) → reconstruct → PAT image at time 1

**Timestep 2**
measure → compressed data from a sub-set (1/N) of patterns → combine → combined data from N previous timesteps (eg. in k-space) → reconstruct → PAT image at time 2

**Timestep N-1**
measure → compressed data from a sub-set (1/N) of patterns → combine → combined data from N previous timesteps (eg. in k-space) → reconstruct → PAT image at time N-1

**Timestep N**
measure → compressed data from a sub-set (1/N) of patterns → combine → combined data from N previous timesteps (eg. in k-space) → reconstruct → PAT image at time N

repeat from the top

time-varying PAT image

**Figure 6**

# DYNAMIC PAT (k-t space sampling)

**Timestep 1**
data from subset of patterns

**Timestep 2**
data from subset of patterns

combined with existing data

**Timestep N**
data from subset of patterns

full data set collected after N time steps

**Timestep N+1**
recollect data from first set of patterns

full data set consisting of previous N timesteps

**Figure 7**

**Figure 8**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6075603 A
- US 7262861 B

**Non-patent literature cited in the description**

- **ZHANG et al.** Backward-mode multi-wavelength photoacoustic scanner using a planar Fabry-Perot polymer film ultrasound sensor for high-resolution three-dimensional imaging of biological tissues. *Applied Optics,* 01 February 2008, vol. 47 (4), 561-577
- **T. ALLEN ; A. HALL ; A.P. DHILLON ; P. BEARD.** *J. Biomed. Opt.,* vol. 17 (6), 2012
- **P. BEARD.** *Interface Focus,* 2011, vol. 1 (4), 602-631
- **J. M. BIOUCAS-DIAS ; M. A. T. FIGUEIREDO.** *IEEE Trans. Im. Proc.,* 2007, vol. 16 (12), 2992-3004
- **A. M. BROOKSTEIN ; D. L. DONOHO ; M. ELAD.** *SIAM Review,* May 2009, vol. 51 (1), 34-81
- **E. CANDES ; L. DEMANET ; D. DONOHO ; L. YING.** *Multiscale Model. Sim.,* 2006, vol. 5 (3), 861-899
- **E. J. CANDES ; L. DEMANET.** *Comm. Pure and Applied Math.,* 2005, vol. 58 (11), 1472-1528
- **E. J. CANDES ; T. TAO.** *IEEE Trans. Inf. Theory,* 2006, vol. 52 (12), 5406-5425
- **E. J. CANDES ; M. B. WAKIN ; S. P. BOYD.** *J. Fourier Anal. Appl.,* 2008, vol. 14 (5-6), 877-905
- **B. T. COX ; S. KARA ; S. R. ARRIDGE ; P. C. BEARD.** *J. Acoust. Soc. Am.,* 2007, vol. 121 (6), 3453-64
- **M. N. DO ; M. VETTERLI.** *IEEE Trans. Im. Proc.,* 2005, vol. 14, 2091-2106
- **D. L. DONOHO ; A. MALEKI ; A. MONTANARI.** *PNAS,* November 2009, vol. 106 (45), 18914-18919
- **M. DUARTE.** Signal Processing Magazine. IEEE, March 2008, vol. 25, 83-91
- **Z. GUO ; C. LI ; L. SONG ; L. V. WANG.** *J. Biomed. Opt.,* 2011, vol. 15 (2), 021311
- **H. JUNG ; J. C. YE ; E. Y. KIM.** *Phys. Med. Biol.,* 2007, vol. 52, 3201-3226
- **J. LAUFER.** *J. Biomed. Opt.,* 2012, vol. 17 (6
- **J. LAUFER.** *J. Biomed. Opt.,* 2012, vol. 17 (5
- **K. P. KOESTLI ; M. FRENZ ; H. BEBIE ; H. P. WEBER.** *Phys. Med. Biol.,* 2001, vol. 46 (7), 1863-72
- **K. KREUTZ-DELGADO.** *Neural Comp.,* 2003, vol. 15, 349-396
- **R. A. KRUGER ; R. B. LAM ; D. R. REINECKE ; S. P. DEL RIO ; R. P. DOYLE.** *Med. Phys.,* 2010, vol. 37 (11), 6096
- **G. KUTYNIOK ; M. SHAHRAM ; X. ZHUANG.** *arXiv.org, math.NA,* June 2011
- **M.LUSTIG ; D. DONOHO ; J. PAULY.** *Magn Reson Med,* 2007, vol. 58, 1182-1195
- **S. PRINCE.** *Phys. Med. Biol.,* 2003, vol. 48 (11), 1491-1504
- **J. PROVOST ; F. LESAGE.** *IEEE Trans. Med. Im.,* 2009, vol. 28 (4), 585-94
- **M. SUN ; N. FENG ; Y. SHEN ; X. SHEN ; L. MA ; J. LI ; Z. WU.** *Optics Exp.,* 2011, vol. 19 (16), 14801-14806
- **J. TANG ; B. E. NETT ; G. H. CHEN.** *Med. Phys.,* 2009, vol. 54, 5781-5804
- **B. E. TREEBY ; B. T. COX.** *J. Biomed. Opt.,* 2010, vol. 15 (2), 021314
- **J. A. TROPP ; A. C. GILBERT.** *IEEE Trans. Inf. Theory,* 2007, vol. 53 (12), 4655-4666
- **J. TSAO ; P. BOESIGER ; K. P. PRUESSMANN.** *Magn Reson Med,* November 2003, vol. 50 (5), 1031-1042
- **G. WANG ; J. QI.** *IEEE Trans. Med. Im.,* 2009, vol. 28 (11), 1717-1726
- **L. WANG.** Photoacoustic Imaging and Spectroscopy. CRC Press, 2009
- **X. WANG ; Y. PANG ; G. KU ; X. XIE ; G. STOCIA ; L. V. WANG.** *Nature Biotechnology,* 2003, vol. 21 (7), 803-806
- **W. YIN ; S. OSHER ; D. GOLDFARB ; J. DARBON.** *SIAM Journal on Imaging Sciences,* 2008, vol. 1 (1), 143-168
- **E. Z. ZHANG ; J. G. LAUFER ; R. B. PEDLEY ; P. C. BEARD.** *Phys. Med. Biol.,* 2009, vol. 54 (4), 1035-46
- **P.C. BEARD.** Review: Biomedical Photoacoustic Imaging. *Interface Focus,* 2001, vol. 1, 602-631
- **M. KLANN ; C KOCH.** *IEEE Trans. on Ultrasonics, Ferroelectrics and Frequency Control,* 2005, vol. 52 (9), 1546-1554
- **K. P. KOESTLI et al.** *Applied Optics,* 2001, vol. 40 (22), 3800-3809

- **V. V. YAKOVLEV et al.** *Advanced Materials, Wiley online library,* 2013, 1-6
- **J.D. HAMILTON et al.** *IEEE Trans. on Ultrasonics, Ferroelectrics and Frequency Control,* 2000, vol. 47 (1), 160-169
- **HUYNH et al.** *Proc. Of SPIE: Photons plus Ultrasound: Imaging and Sensing,* March 2014, vol. 8943, 894327-1